# FASCICULE DE BREVET EUROPEEN

(11) **EP 1 347 737 B1**
(45) Date de publication et mention de la délivrance du brevet: **03.03.2010**
(21) Numéro de dépôt: 02710936.2
(22) Date de dépôt: 02.01.2002
(51) Int. Cl.: A61K 8/41, A61Q 5/12

(54) **COMPOSITION COSMETIQUE COMPRENANT UNE SILICONE VOLATILE, UN TENSIOACTIF SILICONE ET UN TENSIOACTIF CATIONIQUE**
KOSMETISCHE ZUSAMMENSETZUNG MIT EINEM FLÜCHTIGEN SILIKON, EINEM SILIKON-TENSID UND EINEM KATIONISCHEN TENSID
COSMETIC COMPOSITION COMPRISING A VOLATILE SILICONE, A SILICONE SURFACTANT AND A CATIONIC SURFACTANT

(30) Priorité: 02.01.2001 FR 0100015
(43) Date de publication de la demande: 01.10.2003
(73) Titulaire: L'Oréal, 75008 Paris (FR)
(72) Inventeur: FACK, Géraldine, F-92300 Levallois-Perret (FR); GAWTREY, Jonathan, F-92100 Boulogne-Billancout (FR); NICOLAS-MORGANTINI, Luc, F-60810 Rully (FR); RESTLE, Serge, F-95390 Saint-Prix (FR)
(74) Mandataire: Casalonga, Axel
(86) Numéro de dépôt international: PCT/FR2002/000003
(87) Numéro de publication internationale: WO 2002/053112

(56) Documents cités:
- EP-A- 0 331 833
- EP-A- 0 576 748
- EP-A- 0 717 978
- EP-A- 0 782 846
- WO-A-01/00141
- WO-A-01/28506
- WO-A-99/66883

## Description

La présente invention est relative à une composition de traitement cosmétique des matières kératiniques, de type émulsion eau-dans-huile, comprenant dans un milieu cosmétiquement acceptable, au moins une silicone volatile, au moins un tensioactif siliconé et au moins un tensioactif cationique, et à un procédé de traitement cosmétique des matières kératiniques.

Des compositions de soin capillaire sous forme d'émulsions eau-dans-huile comprenant des huiles de silicone et des tensioactifs siliconés sont connues de WO 01/00141 et WO 01/28506.

Les mélanges de silicone volatile et de tensioactif siliconé utilisés pour le traitement des cheveux, et en particulier comme après-shampoing, ne permettent pas d'obtenir des propriétés cosmétiques satisfaisantes. En outre, après rinçage des cheveux, il reste toujours des traces du mélange sur les cheveux.

La demanderesse a découvert de manière surprenante qu'en introduisant un tensioactif cationique du type sel d'ammonium quaternaire de formule (VI) en une concentration strictement supérieure à 0,5 % en poids, dans un mélange de silicone volatile et de tensioactif siliconé, on améliorait nettement les propriétés cosmétiques des cheveux, par exemple, le démêlage et la douceur.

En outre, l'introduction d'un tensioactif cationique dans ce mélange permet d'améliorer la rinçabilité des cheveux.

La présente invention a donc pour objet une composition de traitement des matières kératiniques, de type émulsion eau-dans-huile, comprenant dans un milieu cosmétiquement acceptable, au moins une silicone volatile, au moins un tensioactif siliconé et au moins un tensioactif cationique du type sel d'ammonium quaternaire de formule (VI) en une concentration strictement supérieure à 0,5 % en poids par rapport au poids total de la composition.

Un autre objet de l'invention consiste en un procédé de traitement cosmétique des matières kératiniques mettant en oeuvre une composition selon l'invention telle que décrite ci-dessous.

L'invention a encore pour objet une utilisation de la composition selon l'invention comme après-shampoing.

D'autres objets, caractéristiques, aspects et avantages de l'invention apparaîtront encore plus clairement à la lecture de la description et des divers exemples qui suivent.

Selon l'invention, la composition de traitement cosmétique des matières kératiniques, de type émulsion eau-dans-huile, comprend dans un milieu cosmétiquement acceptable, au moins une silicone volatile, au moins un tensioactif siliconé et au moins un tensioactif cationique tel que défini ci-dessous, en une concentration strictement supérieure à 0,5 % en poids par rapport au poids total de la composition.

La composition selon l'invention comprend une quantité totale d'huiles comprenant au moins ladite silicone volatile, inférieure ou égale à 20 % en poids, de préférence comprise entre 5 et 20 % en poids par rapport au poids total de la composition.

Par huile, on entend dans la présente invention tout corps gras non miscible à l'eau, qui est liquide à température ambiante.

Les huiles utilisables dans la présente invention peuvent comprendre en outre au moins l'un des composés choisis parmi les huiles végétales, les huiles animales, les huiles minérales, les huiles synthétiques, les esters d'acide gras, et leurs mélanges.

Comme huile végétale, on peut notamment mentionner l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum.

Comme huile animale, on peut notamment citer le perhydro-squalène.

Comme huile minérale, on peut notamment citer l'huile de paraffine et l'huile de vaseline.

A titre d'exemples d'huile synthétique, on peut citer le squalane, les poly(α-oléfines) comme l'isododécane ou l'isohexadécane, les huiles végétales transestérifiées, les huiles fluorées, et leurs mélanges.

Comme esters d'acide gras, on peut citer, par exemple, les composés de formule RₐCOOR_{b} dans laquelle Rₐ représente le reste d'un acide gras supérieur comportant de 6 à 29 atomes de carbone et R_{b} représente une chaîne hydrocarbonée contenant de 3 à 30 atomes de carbone, telles que l'huile de Purcellin (octanoate de stéaryle), le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyldécyle, le palmitate de 2-octyldécyle, le myristate ou le lactate de 2-octyl-dodécyle.

La composition selon l'invention présente en particulier une force minimum de résistance à la pénétration de 0,075 N, telle que mesurée par pénétrométrie.

Les mesures de pénétrométrie ont été effectuées avec un analyseur de texture TA-TX2 (Rhéo). Elles correspondent à des mesures de force en compression dans les conditions suivantes :
- déplacement d'un disque (cylindre en ébonite de diamètre 13 mm) à la vitesse de 1 mm/s et détection de la force de résistance à la compression,
- pénétration dans le produit à la même vitesse que ci-dessus sur une profondeur de 10 mm,
- maintien de la compression dans le produit à cette profondeur pendant 300 s, et
- retrait de la sonde et détection de la force de rupture, à la vitesse de 1 mm/s.

Les tensioactifs cationiques utilisables la composition selon l'invention sont choisis parmi les sels d'ammonium quaternaire qui présentent la formule générale (VI) suivante : 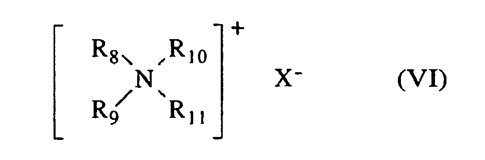 dans laquelle :
R₈ représente un groupe alkyle en C₁₂₋₃₀, de préférence en C₁₄₋₂₂, alcényle en C₁₂₋₃₀, alkyl(C₁₂-C₂₂)amidoalkyle(C₂-C₆), alkyl(C₁₂-C₂₂)-acétate, ou un groupe aromatique tel qu'aryle ou alkylaryle en C₆-C₁₂,
R₉ à R₁₁, qui peuvent être identiques ou différents, représentent un groupe alkyle en C₁₋₈, alcényle en C₁₋₈, alcoxy en C₁₋₈, hydroxyalkyle en C₁₋₈, polyoxyalkylène (C₂-C₆) ou alkylamide en C₁₋₈ ;
X est un anion choisi dans le groupe des halogénures, phosphates, acétates, lactates, alkyl(C₂-C₆)sulfates, alkyl- ou alkylaryl-sulfonates ;

Parmi les sels d'ammonium quaternaire de formule (VI), on préfère d'une part, les chlorures d'alkyltriméthylammonium dans lesquels le radical alkyle comporte environ de 14 à 22 atomes de carbone, en particulier les chlorures de béhényltriméthylammonium, d'arachidyltriméthylammonium, de stéaryltriméthylammonium, de cétyltriméthylammonium, ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL^{®} 70 par la société VAN DYK.

Les tensioactifs cationiques particulièrement préférés dans la composition de l'invention sont choisis parmi le chlorure de béhényltriméthylammonium et le chlorure de palmitylamidopropyltriméthylammonium.

La composition de traitement cosmétique des matières kératiniques comprend de préférence le ou les tensioactifs cationiques en une quantité comprise entre 0,5 et 10 % en poids, mieux encore entre 0,8 et 8 % en poids, et encore plus préférentiellement entre 1 et 5 % en poids par rapport au poids total de la composition.

Les silicones volatiles utilisables dans l'invention sont des silicones linéaires ou cycliques, ayant une viscosité à température ambiante et sous pression atmosphérique, inférieure à 8 mm²/s (8 cSt).

La viscosité est mesurée de préférence par viscosimétrie capillaire, par exemple, à l'aide d'un viscosimètre capillaire, notamment de type Ubbelohde, à une température de 25 °C, selon la norme ASTM D445-97. On peut aussi utiliser la méthode dite de la chute de la bille.

Les silicones volatiles présentent généralement un point d'ébullition compris entre 60 °C et 260° C, et sont plus particulièrement choisies parmi :
(i) les silicones volatiles cycliques comportant de 3 à 7 atomes de silicium et, de préférence, 4 à 5. Il s'agit, par exemple, de l'octaméthylcyclotétrasiloxane commercialisé notamment sous le nom de "VOLATILE SILICONE 7207" par UNION CARBIDE ou "SILBIONE^{®} 70045 V 2" par RHODIA, le décaméthylcyclopentasiloxane commercialisé sous le nom de "VOLATILE SILICONE 7158" par UNION CARBIDE, "SILBIONE^{®} 70045 V 5" par RHODIA ou sous le nom DC245 Fluid par DOW CORNING, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique :
   avec D : 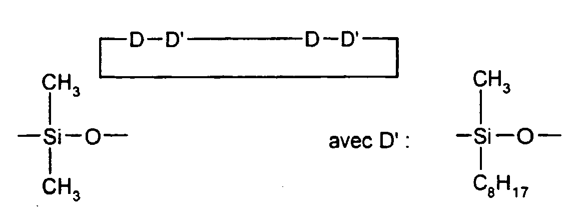
   On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5 mm²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

La composition selon l'invention comprend de préférence les silicones volatiles en une quantité comprise entre 5 et 20 % en poids, et encore plus préférentiellement entre 8 et 15 % en poids par rapport au poids total de la composition.

Les tensioactifs siliconés utilisables dans la présente invention sont ceux bien connus de l'homme du métier. Ils peuvent être hydrosolubles, spontanément hydrodispersibles ou non hydrosolubles. De préférence, ils sont hydrosolubles ou spontanément hydrodispersibles.

Les tensioactifs siliconés sont, par exemple, choisis parmi les composés de formules générales (I), (II), (III), (IV) et (V) : 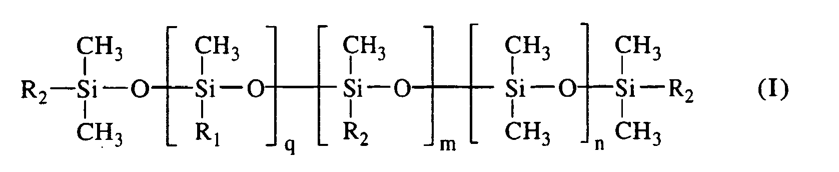 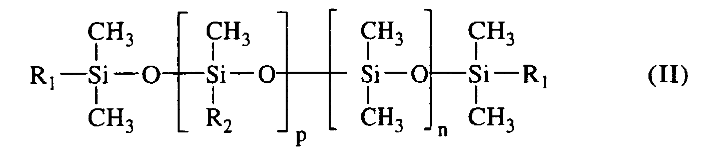 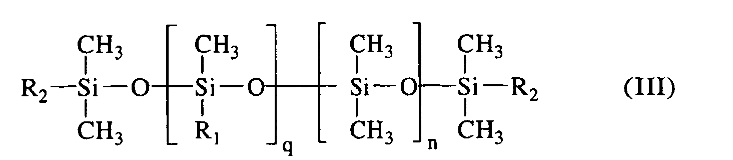 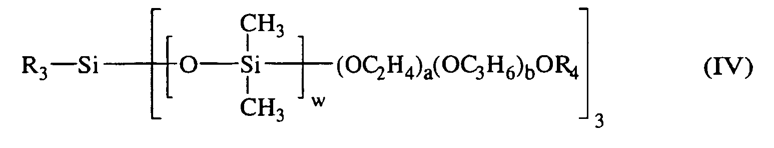 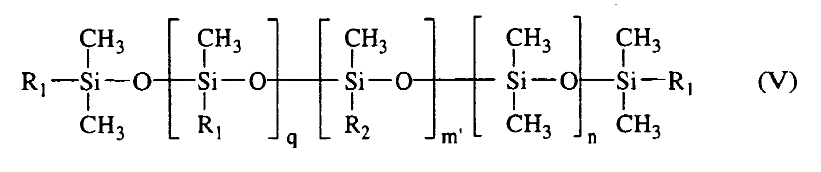 formules dans lesquelles :
- R₁, identique ou différent, représente un groupe alkyle, linéaire ou ramifié, en C₁-C₃₀, ou phényle ;
- R₂, identique ou différent, représente -C_{c}H_{2c}-O-(C₂H₄O)ₐ-(C₃H₆O)_{b}-R₅ ou -C_{c}H_{2c}-O-(C₄H₈O)ₐ-R₅ ;
- R₃ et R₄, identiques ou différents, désignent chacun un groupe alkyle, linéaire ou ramifié, en C₁-C₁₂, et de préférence un groupe méthyle ;
- R₅, identique ou différent, est choisi parmi un atome d'hydrogène, un groupe alkyle, linéaire ou ramifié, comportant de 1 à 12 atomes de carbone, un groupe alcoxy, linéaire ou ramifié, comportant de 1 à 6 atomes de carbone, un groupe acyle, linéaire ou ramifié, comportant de 2 à 12 atomes de carbone, un groupe hydroxyle, -SO₃M, -OCOR₆, aminoalcoxy en C₁-C₆ éventuellement substitué sur l'amine, aminoacyle en C₂-C₆ éventuellement substitué sur l'amine, -NHCH₂CH₂COOM,
- N(CH₂CH₂COOM)₂, aminoalkyle en C₁-C₁₂ éventuellement substitué sur l'amine et sur la chaîne alkyle, carboxyacyle en C₁-C₃₀, un groupement phosphono éventuellement substitué par un ou deux groupes aminoalkyle en C₁-C₁₂ substitués, -CO(CH₂)_{d}COOM, -OCOCHR₇(CH₂)_{d}COOM, -NHCO(CH₂)_{d}OH, -NH₃Y ;
- M, identique ou différent, désigne un atome d'hydrogène, Na, K, Li, NH₄ ou une amine organique ;
- R₆ désigne un groupe alkyle, linéaire ou ramifié, en C₁-C₃₀,
- R₇ désigne un atome d'hydrogène ou un groupe SO₃M ;
- d varie de 1 à 10 ;
- m varie de 0 à 20 ;
- m' varie de 1 à 20 ;
- n varie de 0 à 500 ;
- p varie de 1 à 50 ;
- q varie de 0 à 20 ;
- a varie de 0 à 50 ;
- b varie de 0 à 50 ;
- a + b est supérieur ou égal à 1 ;
- c varie de 0 à 4 ;
- w varie de 1 à 100 ;
- Y représente un anion minéral ou organique monovalent tel qu'un halogénure (chlorure, bromure), un sulfate, ou un carboxylate (acétate, lactate, citrate).

De préférence, on utilise des tensioactifs siliconés répondant aux formules générales (I) ou (II) telles que définies ci-dessus, et plus particulièrement, ceux répondant aux formules (I) ou (II) dans lesquelles au moins l'une des, et de préférence toutes les conditions suivantes sont satisfaites :
- c est égal à 2 ou 3 ;
- R₁ désigne le groupe méthyle ;
- R₅ représente un atome d'hydrogène, un groupe méthyle ou un groupe acétyle et de préférence un atome d'hydrogène ;
- a varie de 1 à 25 et plus particulièrement de 2 à 25 ;
- b varie de 0 à 25, de préférence de 10 à 20 ;
- n varie de 0 à 100 ;
- p varie de 1 à 20.

Les tensioactifs siliconés les plus particulièrement préférés sont, par exemple, ceux vendus sous les dénominations commerciales FLUID DC 193 et DC 5225C par la société DOW CORNING, SILWET^{®} L 77 par la société OSI et MAZIL^{®} 756 par la société MAZER PPG.

Les tensioactifs siliconés sont contenus dans la présente invention en une quantité comprise entre 0,01 et 3 % en poids, mieux encore comprise entre 0,2 et 3 % en poids par rapport au poids total de la composition de traitement des matières kératiniques.

Par "milieu cosmétiquement acceptable", on entend un milieu compatible avec toutes les matières kétatiniques telles que la peau, les cheveux, les ongles, les cils, les sourcils, les lèvres et toute autre zone du corps et du visage, mais aussi d'odeur, d'aspect et de toucher agréables.

Le milieu aqueux cosmétiquement acceptable comprend l'eau ou un mélange d'eau et d'un solvant cosmétiquement acceptable choisi parmi les alcools inférieurs en C₁-C₄, tels que l'éthanol, l'isopropanol, le tertio-butanol ou le n-butanol ; les polyols comme le propylèneglycol ; les éthers de polyols ; les alcanes en C₅-C₁₀ ; les cétones en C₃₋₄ comme l'acétone et la méthyléthylcétone ; les acétates d'alkyle en C₁-C₄ comme l'acétate de méthyle, l'acétate d'éthyle et l'acétate de butyle ; le diméthoxyéthane, le diéthoxyéthane ; et leurs mélanges.

La proportion d'eau dans la composition selon l'invention est comprise de préférence entre 30 et 90 % en poids par rapport au poids total de la composition.

Le pH des compositions de l'invention est compris entre 4 et 8, de préférence entre 5 et 7.

Les compositions selon l'invention peuvent également contenir des additifs tels que des polymères cationiques, anioniques, non ioniques ou amphotères, des silicones non volatiles, modifiées ou non, des épaississants polymériques naturels ou synthétiques, anioniques, amphotères, zwittérioniques, non ioniques ou cationiques, associatifs ou non, des épaississants non polymériques comme des électrolytes, des sucres, des nacrants, des opacifiants, des filtres solaires, des parfums, des colorants, des particules organiques ou minérales, des conservateurs, des agents de stabilisation du pH.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 50 % en poids par rapport au poids total de la composition.

Les compositions selon l'invention peuvent se présenter sous forme de liquides fluides ou épaissis, de gels, de crèmes, ou d'émulsions simples ou multiples.

Les compositions peuvent être utilisées, par exemple, comme shampoings, produits de coloration ou de décoloration ou de permanente, produits de coiffage, soins rincés, masques de soin profond, gels douches, lotions ou crèmes de traitement du cuir chevelu, produits de rasage ou produits d'épilation.

La présente invention concerne également un procédé de traitement cosmétique des matières kératiniques qui consiste à appliquer une quantité efficace d'une composition telle que décrite ci-dessus, sur les matières kératiniques, à effectuer un éventuel rinçage après un éventuel temps de pose.

Selon un mode de réalisation préféré de l'invention, la composition peut être utilisée comme après-shampoing.

Les exemples suivants illustrent la présente invention et ne doivent être considérés en aucune manière comme limitant l'invention.

### EXEMPLES

On a préparé des compositions d'après-shampoing à partir des ingrédients indiqués dans le tableau ci-dessous. Les teneurs indiquées sont exprimées en % en poids par rapport au poids total de la composition.

| | | Ex. 1 | Ex. 2 | Ex. 3 | Ex. 4 |
|---|---|---|---|---|---|
| Cyclopentadiméthylsiloxane⁽¹⁾ | | 8,1 | 7 | 5,4 | 10,1 |
| Myristate d'isopropyle | | - | - | 2,1 | - |
| Polyaminosiloxane | | - | - | - | 2 |
| Polydiméthyl/méthyl(396/4)siloxane (18 moles d'oxyde d'éthylène/ 18 moles d'oxyde de propylène)⁽²⁾ - DC 5225C de DOW CORNING | | 0,9 MA | 0,5 MA | 0,5 MA | 0,5 MA |
| Chlorure de palmitylamidopropyltriméthylammonium | | - | 1,2 | 2,7 | 1,2 |
| Chlorure de béhényltriméthylammonium | | 3,2 | - | - | - |
| Propylèneglycol | | - | 0,8 | 7,3 | 2,5 |
| Saccharose | | - | - | 29,5 | - |
| Glycérine | | - | 40,8 | - | - |
| Filtre benzotriazole dérivé de l'heptaméthylhydrogénotrisiloxane | | - | - | - | 1 |
| Eau | qsp | 100 | 100 | 100 | 100 |

| | | | | | |
|---|---|---|---|---|---|
| MA : Matières actives ⁽¹⁾ DC 245 de DOW CORNING ⁽²⁾ à 10 % dans du cyclopentadiméthylsiloxane. | | | | | |

On a appliqué les compositions sur les cheveux et rincé après un temps de pose d'une minute, le rinçage étant aisé. On a ensuite fait séché les cheveux.

Les cheveux séchés sont doux et déliés au toucher, et sans résidus désagréables.

## Revendications

1. Composition de traitement cosmétique des matières kératiniques de type émulsion eau-dans-huile, **caractérisée en ce qu'**elle comprend dans un milieu cosmétiquement acceptable, au moins une silicone volatile, au moins un tensioactif siliconé et au moins un tensioactif cationique en une concentration strictement supérieure à 0,5 % en poids par rapport au poids total de la composition, choisi parmi les sels d'ammonium quaternaire,
lesdits sels d'ammonium quaternaire étant choisis parmi :
- ceux qui présentent la formule générale (VI) suivante : 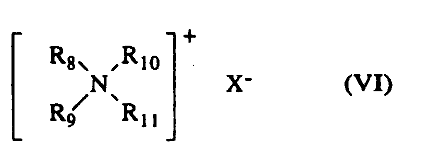
dans laquelle :
R₈ représente un groupe alkyle en C₁₂₋₃₀, de préférence en C₁₄₋₂₂, alcényle en C₁₂₋₃₀, alkyl(C₁₂-C₂₂)amidoalkyle(C₂-C₆), alkyl(C₁₂-C₂₂)-acétate, ou un groupe aromatique tel qu'aryle ou alkylaryle en C₆-C₁₂,
R₉ à R₁₁, qui peuvent être identiques ou différents, représentent un groupe alkyle en C₁₋₈, alcényle en C₁₋₈, alcoxy en C₁₋₈, hydroxyalkyle en C₁₋₈, polyoxyalkylène (C₂-C₆) ou alkylamide en C₁₋₈,
X est un anion choisi dans le groupe des halogénures, phosphates, acétates, lactates, alkyl(C₂-C₆)sulfates, alkyl- ou alkylaryl-sulfonates ;
la quantité totale d'huiles, qui comprend au moins ladite silicone volatile, étant inférieure ou égale à 20 % en poids par rapport au poids total de la composition.

2. Composition de traitement cosmétique des matières kératiniques selon la revendication 1, **caractérisée en ce que** les huiles comprennent en outre au moins l'un des composés choisis parmi les huiles végétales, les huiles animales, les huiles minérales, les huiles synthétiques, les esters d'acide gras, et leurs mélanges.

3. Composition selon la revendication 1 ou 2, **caractérisée en ce que** la quantité d'huiles est comprise entre 5 et 20 % en poids par rapport au poids total de la composition.

4. Composition de traitement cosmétique des matières kératiniques selon l'une quelconque des revendications précédentes, **caractérisée en ce que** les tensioactifs cationiques sont choisis parmi le chlorure de béhényltriméthylammonium et le chlorure de palmitylamidopropyltriméthylammonium.

5. Composition de traitement cosmétique des matières kératiniques selon l'une quelconque des revendications précédentes, **caractérisée en ce qu'**elle comprend les tensioactifs cationiques en une quantité comprise entre 0,5 et 10 % en poids par rapport au poids total de la composition.

6. Composition de traitement cosmétique des matières kératiniques selon la revendication 5, **caractérisée en ce qu'**elle comprend les tensioactifs cationiques en une quantité comprise entre 0,8 et 8 % en poids par rapport au poids total de la composition.

7. Composition de traitement cosmétique des matières kératiniques selon la revendication 6, **caractérisée en ce qu'**elle comprend les tensioactifs cationiques en une quantité comprise entre 1 et 5 % en poids, par rapport au poids total de la composition.

8. Composition de traitement cosmétique des matières kératiniques selon l'une quelconque des revendications précédentes, **caractérisée en ce que** les silicones volatiles sont des silicones linéaires ou cycliques, ayant une viscosité à température ambiante et sous pression atmosphérique, inférieure à 8 mm²/s.

9. Composition de traitement cosmétique des matières kératiniques selon la revendication 8, **caractérisée en ce que** les silicones volatiles sont choisies parmi i) les silicones volatiles cycliques comportant de 3 à 7 atomes de silicium, et ii) les silicones volatiles linéaires ayant 2 à 9 atomes de silicium et possédant une viscosité inférieure ou égale à 5 mm²/s à 25 °C.

10. Composition de traitement cosmétique des matières kératiniques selon l'une quelconque des revendications précédentes, **caractérisée en ce qu'**elle comprend les silicones volatiles en une quantité comprise entre 5 et 20 % en poids, par rapport au poids total de la composition.

11. Composition de traitement cosmétique des matières kératiniques selon la revendication 10, **caractérisée en ce qu'**elle comprend les silicones volatiles en une quantité comprise entre 8 et 15 % en poids, par rapport au poids total de la composition.

12. Composition de traitement cosmétique des matières kératiniques selon l'une quelconque des revendications précédentes, **caractérisée en ce** les tensioactifs siliconés sont choisis parmi les composés de formules générales (I), (II), (III), (IV) et (V): 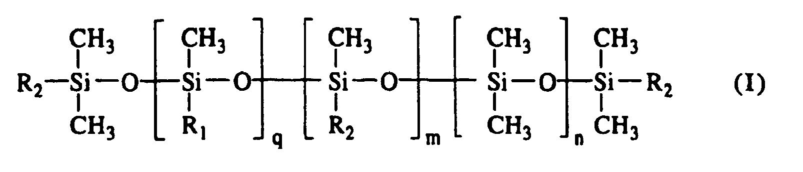 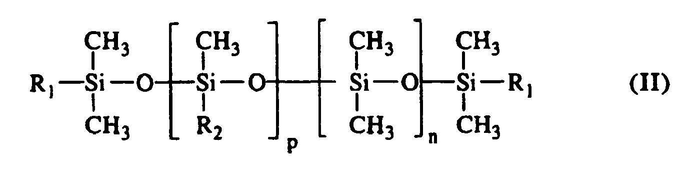 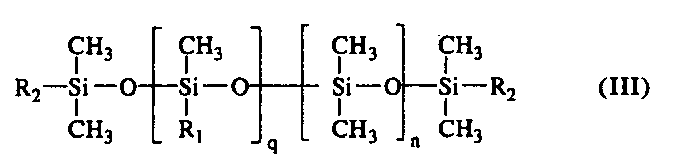 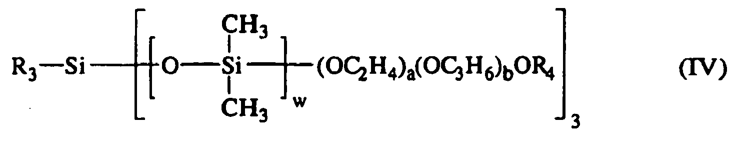 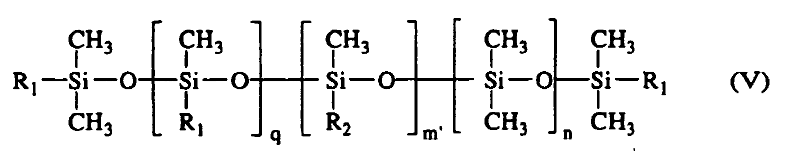 formules dans lesquelles :
- R₁, identique ou différent, représente un groupe alkyle, linéaire ou ramifié, en C₁-C₃₀, ou phényle ;
- R₂, identique ou différent, représente -C_{c}H_{2c}-O-(C₂H₄O)ₐ-(C₃H₆O)_{b}-R₅ ou -C_{c}H_{2c}-O-(C₄H₈O)ₐ-R₅ ;
- R₃ et R₄, identiques ou différents, désignent chacun un groupe alkyle, linéaire ou ramifié, en C₁-C₁₂, et de préférence un groupe méthyle ;
- R₅, identique ou différent, est choisi parmi un atome d'hydrogène, un groupe alkyle, linéaire ou ramifié, comportant de 1 à 12 atomes de carbone, un groupe alcoxy, linéaire ou ramifié, comportant de 1 à 6 atomes de carbone, un groupe acyle, linéaire ou ramifié, comportant de 2 à 12 atomes de carbone, un groupe hydroxyle, -SO₃M, -OCOR₆, aminoalcoxy en C₁-C₆ éventuellement substitué sur l'amine, aminoacyle en C₂-C₆, éventuellement substitué sur l'amine, -NHCH₂CH₂COOM,
- N(CH₂CH₂COOM)₂, aminoalkyle en C₁-C₁₂ éventuellement substitué sur l'amine et sur la chaîne alkyle, carboxyacyle en C₁-C₃₀, un groupement phosphono éventuellement substitué par un ou deux groupes aminoalkyle en C₁-C₁₂ substitués, -CO(CH₂)_{d}COOM, -OCOCHR₇(CH₂)_{d}COOM, -NHCO(CH₂)_{d}OH, -NH₃Y ;
- M, identique ou différent, désigne un atome d'hydrogène, Na, K, Li, NH₄ ou une amine organique ;
- R₆ désigne un groupe alkyle, linéaire ou ramifié, en C₁-C₃₀ ;
- R₇ désigne un atome d'hydrogène ou un groupe SO₃M ;
- d varie de 1 à 10 ;
- m varie de 0 à 20 ;
- m' varie de 1 à 20 ;
- n varie de 0 à 500 ;
- p varie de 1 à 50 ;
- q varie de 0 à 20 ;
- a varie de 0 à 50 ;
- b varie de 0 à 50 ;
- a + b est supérieur ou égal à 1 ;
- c varie de 0 à 4 ;
- w varie de 1 à 100 ;
- Y représente un anion minéral ou organique monovalent tel qu'un halogénure, un sulfate, ou un carboxylate.

13. Composition de traitement cosmétique des matières kératiniques selon la revendication 12, **caractérisée en ce** les tensioactifs siliconés sont choisis parmi ceux répondant aux formules (I) ou (II) dans lesquelles au moins l'une des, et de préférence toutes les conditions suivantes sont satisfaites :
- c est égal à 2 ou 3 ;
- R₁ désigne le groupe méthyle ;
- R₅ représente un atome d'hydrogène, un groupe méthyle ou un groupe acétyle et de préférence un atome d'hydrogène ;
- a varie de 1 à 25 et plus particulièrement de 2 à 25 ;
- b varie de 0 à 25 ;
- n varie de 0 à 100 ;
- p varie de 1 à 20.

14. Composition de traitement cosmétique des matières kératiniques selon l'une quelconque des revendications précédentes, **caractérisée en ce qu'**elle comprend les tensioactifs siliconés en une quantité comprise entre 0,01 et 3 % en poids par rapport au poids total de la composition.

15. Composition de traitement cosmétique des matières kératiniques selon la revendication 14, **caractérisée en ce qu'**elle comprend les tensioactifs siliconés en une quantité comprise entre 0,2 et 3 % en poids, par rapport au poids total de la composition.

16. Composition de traitement cosmétique des matières kératiniques selon l'une quelconque des revendications précédentes, **caractérisée en ce que** le milieu cosmétiquement acceptable comprend l'eau ou un mélange d'eau et d'un solvant cosmétiquement acceptable.

17. Composition de traitement cosmétique des matières kératiniques selon la revendication 16, **caractérisée en ce que** le solvant cosmétiquement acceptable est choisi parmi les alcools inférieurs en C₁-C₄, les polyols, les éthers de polyols, les alcanes en C₅-C₁₀, les cétones en C₃₋₄, les acétates d'alkyle en C₁-C₄, le diméthoxyéthane, le diéthoxyéthane, et leurs mélanges.

18. Composition de traitement cosmétique des matières kératiniques selon l'une quelconque des revendications précédentes, **caractérisée en ce qu'**elle comprend des additifs tels que des polymères cationiques, anioniques, non ioniques ou amphotères, des silicones non volatiles, modifiées ou non, des épaississants polymériques naturels ou synthétiques, anioniques, amphotères, zwittérioniques, non ioniques ou cationiques, associatifs ou non, des épaississants non polymériques comme des électrolytes, des sucres, des nacrants, des opacifiants, des filtres solaires, des parfums, des colorants, des particules organiques ou minérales, des conservateurs, des agents de stabilisation du pH.

19. Procédé de traitement cosmétique des matières kératiniques, **caractérisé en ce que** l'on applique sur les matières kératiniques une composition de traitement cosmétique selon l'une quelconque des revendications précédentes.

20. Utilisation d'une composition de traitement cosmétique selon l'une quelconque des revendications 1 à 18, comme après-shampoing.

## Claims

1. Composition for the cosmetic treatment of keratin materials, of water-in-oil emulsion type, **characterized in that** it comprises, in a cosmetically acceptable medium, at least one volatile silicone, at least one silicone surfactant and at least one cationic surfactant in a concentration strictly greater than 0.5% by weight relative to the total weight of the composition, chosen from quaternary ammonium salts, the said quaternary ammonium salts being chosen from:
- those having the general formula (VI) below: 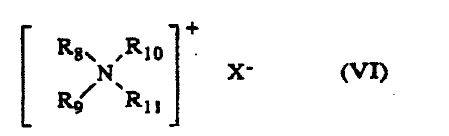
in which:
R₈ represents a C₁₂₋₃₀ and preferably C₁₄₋₂₂ alkyl group, a C₁₂₋₃₀ alkenyl group, a (C₁₂-C₂₂) alkylamido (C₂-C₆) alkyl group, a (C₁₂-C₂₂)alkylacetate group, or an aromatic group such as C₆-C₁₂ aryl or alkylaryl,
R₉ to R₁₁, which may be identical or different, represent a C₁₋₈ alkyl, C₁₋₈ alkenyl, C₁₋₈ alkoxy, C₁₋₈ hydroxyalkyl, polyoxy(C₂-C₆)alkylene or C₁₋₈ alkylamide group,
X is an anion chosen from halides, phosphates, acetates, lactates, (C₂-C₆)alkyl sulfates and alkyl- or alkylaryl- sulfonates;
the total amount of oils, which includes at least the said volatile silicone, being less than or equal to 20% by weight relative to the total weight of the composition.

2. Composition for the cosmetic treatment of keratin materials according to Claim 1, **characterized in that** the oils also comprise at least one of the compounds chosen from plant oils, animal oils, mineral oils, synthetic oils and fatty acid esters, and mixtures thereof.

3. Composition according to Claim 1 or 2, **characterized in that** the amount of oils is between 5% and 20% by weight relative to the total weight of the composition.

4. Composition for the cosmetic treatment of keratin materials according to any one of the preceding claims, **characterized in that** the cationic surfactants are chosen from behenyltrimethylammonium chloride and palmitylamidopropyltrimethylammonium chloride.

5. Composition for the cosmetic treatment of keratin materials according to any one of the preceding claims, **characterized in that** it comprises the cationic surfactants in an amount of between 0.5% and 10% by weight relative to the total weight of the composition.

6. Composition for the cosmetic treatment of keratin materials according to Claim 5, **characterized in that** it comprises the cationic surfactants in an amount of between 0.8% and 8% by weight relative to the total weight of the composition.

7. Composition for the cosmetic treatment of keratin materials according to Claim 6, **characterized in that** it comprises the cationic surfactants in an amount of between 1% and 5% by weight relative to the total weight of the composition.

8. Composition for the cosmetic treatment of keratin materials according to any one of the preceding claims, **characterized in that** the volatile silicones are linear or cyclic silicones, with a viscosity at room temperature and at atmospheric pressure of less than 8 mm²/s.

9. Composition for the cosmetic treatment of keratin materials according to Claim 8, **characterized in that** the volatile silicones are chosen from i) cyclic volatile silicones comprising from 3 to 7 silicon atoms, and ii) linear volatile silicones containing 2 to 9 silicon atoms and having a viscosity of less than or equal to 5 mm²/s at 25°C.

10. Composition for the cosmetic treatment of keratin materials according to any one of the preceding claims, **characterized in that** it comprises the volatile silicones in an amount of between 5% and 20% relative to the total weight of the composition.

11. Composition for the cosmetic treatment of keratin materials according to Claim 10, **characterized in that** it comprises the volatile silicones in an amount of between 8% and 15% by weight relative to the total weight of the composition.

12. Composition for the cosmetic treatment of keratin materials according to any one of the preceding claims, **characterized in that** the silicone surfactants are chosen from the compounds of general formulae (I), (II), (III), (IV) and (V): 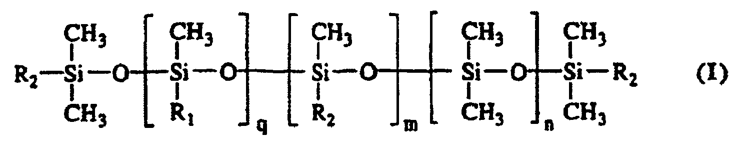 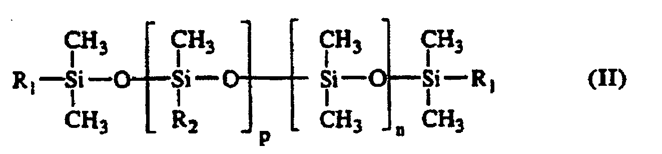 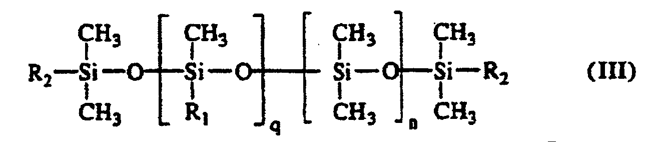 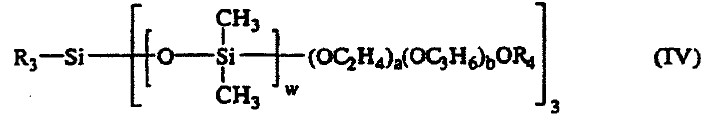 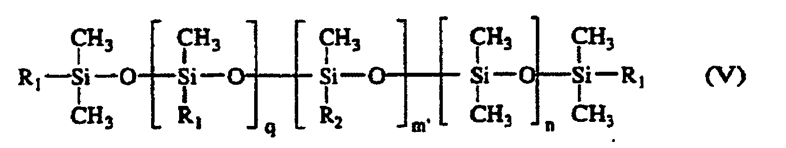 in which formulae:
- R₁, which may be identical or different, represents a linear or branched C₁-C₃₀ alkyl group, or phenyl;
- R₂, which may be identical or different, represents -C_{c}H_{2c}-O-(C₂H₄O)ₐ-(C₃H₆O)_{b}-R₅ or -C_{c}H_{2c}-O-(C₄H₈O)ₐ-R₅;
- R₃ and R₄, which may be identical or different, each denote a linear or branched C₁-C₁₂ alkyl group, and preferably a methyl group;
- R₅, which may be identical or different, is chosen from a hydrogen atom, a linear or branched alkyl group containing from 1 to 12 carbon atoms, a linear or branched alkoxy group containing from 1 to 6 carbon atoms, a linear or branched acyl group containing from 2 to 12 carbon atoms, a hydroxyl group, -SO₃M, -OCOR₆, C₁-C₆ aminoalkoxy optionally substituted on the amine, C₂-C₆ aminoacyl optionally substituted on the amine, -NHCH₂CH₂COOM, -N(CH₂CH₂COOM)₂, C₁-C₁₂ aminoalkyl optionally substituted on the amine and on the alkyl chain, C₁-C₃₀ carboxyacyl, a phosphono group optionally substituted with one or two substituted C₁-C₁₂ aminoalkyl groups, -CO(CH₂)_{d}COOM, -OCOCHR₇(CH₂)_{d}COOM, -NHCO(CH₂)_{d}OH, -NH₃Y;
- M, which may be identical or different, denotes a hydrogen atom, Na, K, Li, NH₄ or an organic amine;
- R₆ denotes a linear or branched C₁-C₃₀ alkyl group;
- R₇ denotes a hydrogen atom or a group SO₃M;
- d ranges from 1 to 10;
- m ranges from 0 to 20;
- m' ranges from 1 to 20;
- n ranges from 0 to 500;
- p ranges from 1 to 50;
- q ranges from 0 to 20;
- a ranges from 0 to 50;
- b ranges from 0 to 50;
- a + b is greater than or equal to 1;
- c ranges from 0 to 4;
- w ranges from 1 to 100;
- Y represents a monovalent mineral or organic anion such as a halide, a sulfate or a carboxylate.

13. Composition for the cosmetic treatment of keratin materials according to Claim 12, **characterized in that** the silicone surfactants are chosen from those corresponding to formula (I) or (II) in which at least one and preferably all of the following conditions are satisfied:
- c is equal to 2 or 3;
- R₁ denotes a methyl group;
- R₅ represents a hydrogen atom, a methyl group or an acetyl group and preferably a hydrogen atom;
- a ranges from 1 to 25 and more particularly from 2 to 25;
- b ranges from 0 to 25;
- n ranges from 0 to 100;
- p ranges from 1 to 20.

14. Composition for the cosmetic treatment of keratin materials according to any one of the preceding claims, **characterized in that** it comprises the silicone surfactants in an amount of between 0.01% and 3% by weight relative to the total weight of the composition.

15. Composition for the cosmetic treatment of keratin materials according to Claim 14, **characterized in that** it comprises the silicone surfactants in an amount of between 0.2% and 3% by weight relative to the total weight of the composition.

16. Composition for the cosmetic treatment of keratin materials according to any one of the preceding claims, **characterized in that** the cosmetically acceptable medium comprises water or a mixture of water and of a cosmetically acceptable solvent.

17. Composition for the cosmetic treatment of keratin materials according to Claim 16, **characterized in that** the cosmetically acceptable solvent is chosen from C₁-C₄ lower alcohols, polyols, polyol ethers, C₅-C₁₀ alkanes, C₃₋₄ ketones, C₁-C₄ alkyl acetates, dimethoxyethane and diethoxyethane, and mixtures thereof.

18. Composition for the cosmetic treatment of keratin materials according to any one of the preceding claims, **characterized in that** it comprises additives such as cationic, anionic, nonionic or amphoteric polymers, modified or unmodified non-volatile silicones, natural or synthetic, anionic, amphoteric, zwitterionic, nonionic or cationic associative or non-associative polymeric thickeners, non-polymeric thickeners, for instance electrolytes, sugars, nacreous agents, opacifiers, sunscreens, fragrances, colorants, organic or mineral particles, preserving agents and pH stabilizers.

19. Process for the cosmetic treatment of keratin materials, **characterized in that** a cosmetic treatment composition according to any one of the preceding claims is applied to the keratin materials.

20. Use of a cosmetic treatment composition according to any one of Claims 1 to 18, as a hair conditioner.

## Patentansprüche

1. Zusammensetzung zur kosmetischen Behandlung von Keratinmaterialien vom Typ Wasser-in-Öl-Emulsion, **dadurch gekennzeichnet, dass** sie in einem kosmetisch verträglichen Medium mindestens ein flüchtiges Silikon, mindestens ein Silikon-Tensid und mindestens ein kationisches Tensid in einer Konzentration von exakt mehr als 0,5 Gew.-%, bezogen auf des Gesamtgewicht der Zusammensetzung, ausgewählt aus den quarternären Ammoniumsalzen, umfasst,
wobei die quarternären Ammoniumsalze ausgewählt sind aus:
- jenen, die die folgende allgemeine Formel (VI) aufweisen: 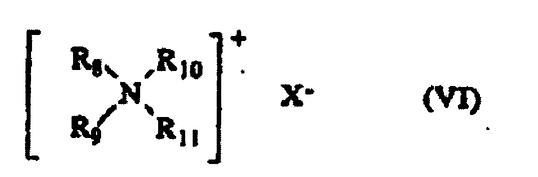
worin:
R₈ für eine C₁₂₋₃₀-Alkyl-, bevorzugt eine C₁₄₋₂₂-Alkyl-, C₁₂₋₃₀-Alkenyl-, (C₁₂-C₂₂) -Alkylamido- (C₂-C₆)-alkyl-, (C₁₂-C₂₂)-Alkylacetatgruppe oder eine aromatische Gruppe, wie etwa C₆-C₁₂-Aryl- oder - Alkylaryl steht,
R₉ bis R₁₁, die gleich oder verschieden sein können, für eine C₁₋₈-Alkyl-, C₁₋₈-Alkenyl-, C₁₋₈-Alkoxy-, C₁₋₈-Hydroxyalkyl-, (C₂-C₆)-Polyoxyalkylen- oder C₁₋₈-Alkylamidgruppe stehen,
X ein Anion ist, ausgewählt aus der Gruppe der Halogenide, Phosphate, Acetate, Lactate, (C₂-C₆)-Alkylsulfate, Alkyl- oder Alkylarylsulfonate;
wobei die Gesamtmenge an Ölen, die mindestens das flüchtige Silikon umfasst, bezogen auf das Gesamtgewicht der Zusammensetzung kleiner oder gleich 20 Gew.-% ist.

2. Zusammensetzung zur kosmetischen Behandlung von Keratinmaterialien nach Anspruch 1, **dadurch gekennzeichnet, dass** die Öle ferner mindestens eine der Verbindungen umfassen, die aus Pflanzenölen, Tierölen, Mineralölen, synthetischen Ölen, Fettsäureestern und deren Gemischen ausgewählt sind.

3. Zusammensetzung nach Anspruch 1 oder 2, **dadurch gekennzeichnet, dass** die Menge an Ölen im Bereich zwischen 5 und 20 Gew.-%, bezogen auf das Gesamtgewicht der Zusammensetzung, liegt.

4. Zusammensetzung zur kosmetischen Behandlung von Keratinmaterialien nach irgendeinem der vorhergehenden Ansprüche, **dadurch gekennzeichnet, dass** die kationischen Tenside aus Behenyltrimethylammoniumchlorid und Palmitylamidopropyltrimethylammoniumchlorid ausgewählt sind.

5. Zusammensetzung zur kosmetischen Behandlung von Keratinmaterialien nach irgendeinem der vorhergehenden Ansprüche, **dadurch gekennzeichnet, dass** sie kationische Tenside in einer Menge im Bereich zwischen 0,5 und 10 Gew.-%, bezogen auf das Gesamtgewicht der Zusammensetzung, umfasst.

6. Zusammensetzung zur kosmetischen Behandlung von Keratinmaterialien nach Anspruch 5, **dadurch gekennzeichnet, dass** sie kationische Tenside in einer Menge im Bereich zwischen 0,8 und 8 Gew.-%, bezogen auf das Gesamtgewicht der Zusammensetzung, umfasst.

7. Zusammensetzung zur kosmetischen Behandlung von Keratinmaterialien nach Anspruch 6, **dadurch gekennzeichnet, dass** sie kationische Tenside in einer Menge im Bereich zwischen 1 und 5 Gew.-%, bezogen auf das Gesamtgewicht der Zusammensetzung, umfasst.

8. Zusammensetzung zur kosmetischen Behandlung von Keratinmaterialien nach irgendeinem der vorhergehenden Ansprüche, **dadurch gekennzeichnet, dass** die flüchtigen Silikone lineare oder cyclische Silikone sind, die bei Umgebungstemperatur und unter Atmosphärendruck eine Viskosität von weniger als 8 mm²/s aufweisen.

9. Zusammensetzung zur kosmetischen Behandlung von Keratinmaterialien nach Anspruch 8, **dadurch gekennzeichnet, dass** die flüchtigen Silikone ausgewählt sind aus i) den cyclischen flüchtigen Silikonen, die 3 bis 7 Siliciumatome umfassen, und ii) den linearen flüchtigen Silikonen, die 2 bis 9 Siliciumatome aufweisen und bei 25 °C eine Viskosität von weniger oder gleich 5 mm²/s besitzen.

10. Zusammensetzung zur kosmetischen Behandlung von Keratinmaterialien nach irgendeinem der vorhergehenden Ansprüche, **dadurch gekennzeichnet, dass** sie flüchtige Silikone in einer Menge im Bereich zwischen 5 und 20 Gew.-%, bezogen auf das Gesamtgewicht der Zusammensetzung, umfasst.

11. Zusammensetzung zur kosmetischen Behandlung von Keratinmaterialien nach Anspruch 10, **dadurch gekennzeichnet, dass** sie flüchtige Silikone in einer Menge im Bereich zwischen 8 und 15 Gew.-%, bezogen auf das Gesamtgewicht der Zusammensetzung, umfasst.

12. Zusammensetzung zur kosmetischen Behandlung von Keratinmaterialien nach irgendeinem der vorhergehenden Ansprüche, **dadurch gekennzeichnet, dass** die Silikon-Tenside aus den Verbindungen mit den allgemeinen Formeln (I), (II), (III), (IV) und (V) ausgewählt sind: 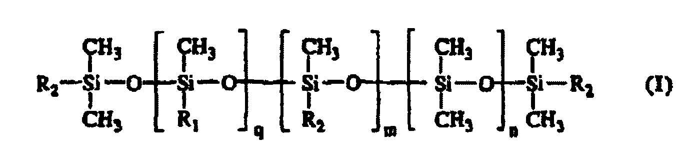 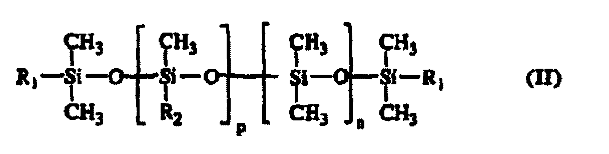 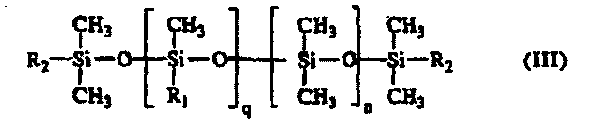 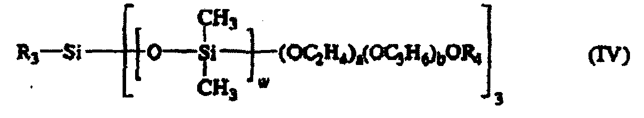 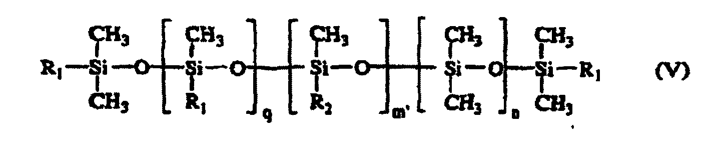 worin in den Formeln:
- R₁, gleich oder verschieden, für eine lineare oder verzweigte C₁-C₃₀-Alkyl- oder Phenylgruppe steht
- R₂, gleich oder verschieden, für -C_{c}H_{2c}-O-(C₂H₄O)ₐ-(C₃H₆O)_{b}-R₅ oder -C_{c}H_{2c}-O-(C₄H₈O)ₐ-R₅ steht;
- R₃ und R₄, gleich oder verschieden, jeweils eine lineare oder verzweigte, C₁-C₁₂-Alkylgruppe und bevorzugt eine Methylgruppe bezeichnen;
- R₅, gleich oder verschieden, ausgewählt ist aus einem Wasserstoffatom, einer linearen oder verzweigten Alkylgruppe, die 1 bis 12 Kohlenstoffatome umfasst, einer linearen oder verzweigten Alkoxygruppe, die 1 bis 6 Kohlenstoffatome umfasst, einer linearen oder verzweigten Acylgruppe, die 2 bis 12 Kohlenstoffatome umfasst, einer Hydroxylgruppe, -SO₃M, -OCOR₆, C₁-C₆-Aminoalcoxy, gegebenenfalls auf dem Amin substituiert, C₂-C₆Aminoacyl, gegebenenfalls auf dem Amin substituiert, -NHCH₂CH₂COOM,-N (CH₂CH₂COOM)₂, C₁-C₁₂-Aminoalkyl, gegebenenfalls auf dem Amin und auf der Alkylkette substituiert, C₁-C₃₀-Carboxyacyl, einem Phosphono-Motiv, gegebenenfalls mit einer oder zwei substituierten C₁-C₁₂ Aminoalkylgruppen substituiert,-CO(CH₂)_{d}COOM, -OCOCHR₇(CH₂)_{d}COOM,-NHCO(CH₂)_{d}OH, -NH₃Y;
- M, gleich oder verschieden, ein Wasserstoffatom, Na, K, Li, NH₄ oder ein organisches Amin bezeichnet;
- R₆ eine lineare oder verzweigte C₁-C₃₀-Alkylgruppe bezeichnet;
- R₇ ein Wasserstoffatom oder eine SO₃M-Gruppe bezeichnet;
- d zwischen 1 und 10 liegt;
- m zwischen 0 und 20 liegt;
- m' zwischen 1 und 20 liegt;
- n zwischen 0 und 500 liegt;
- p zwischen 1 und 50 liegt;
- q zwischen 0 und 20 liegt;
- a zwischen 0 und 50 liegt;
- b zwischen 0 und 50 liegt;
- a + b größer oder gleich 1 ist;
- c zwischen 0 und 4 liegt;
- w zwischen 1 und 100 liegt;
- Y für ein mineralisches oder organisches einwertiges Anion, wie etwa ein Halogenid, ein Sulfat oder ein Carboxylat, steht.

13. Zusammensetzung zur kosmetischen Behandlung von Keratinmaterialien nach Anspruch 12, **dadurch gekennzeichnet, dass** die Silikon-Tenside aus jenen ausgewählt sind, die den Formeln (I) oder (II) entsprechen, worin mindestens eine der und bevorzugt alle folgenden Bedingungen erfüllt sind:
- c gleich 2 oder 3;
- R₁ bezeichnet die Methylgruppe;
- R₅ steht für ein Wasserstoffatom, eine Methylgruppe oder eine Acetylgruppe und bevorzugt ein Wasserstoffatom;
- a liegt zwischen 1 und 25 und insbesondere zwischen 2 und 25;
- b liegt zwischen 0 und 25;
- n liegt zwischen 0 und 100;
- p zwischen 1 und 20 liegt.

14. Zusammensetzung zur kosmetischen Behandlung von Keratinmaterialien nach irgendeinem der vorhergehenden Ansprüche, **dadurch gekennzeichnet, dass** sie Silikon-Tenside in einer Menge im Bereich zwischen 0,01 und 3 Gew.-%, bezogen auf das Gesamtgewicht der Zusammensetzung, umfasst.

15. Zusammensetzung zur kosmetischen Behandlung von Keratinmaterialien nach Anspruch 14, **dadurch gekennzeichnet, dass** sie Silikon-Tenside in einer Menge im Bereich zwischen 0,2 und 3 Gew.-%, bezogen auf das Gesamtgewicht der Zusammensetzung, umfasst.

16. Zusammensetzung zur kosmetischen Behandlung von Keratinmaterialien nach irgendeinem der vorhergehenden Ansprüche, **dadurch gekennzeichnet, dass** das kosmetisch verträgliche Medium Wasser oder ein Gemisch aus Wasser und einem kosmetisch verträglichen Lösemittel umfasst.

17. Zusammensetzung zur kosmetischen Behandlung von Keratinmaterialien nach Anspruch 16, **dadurch gekennzeichnet, dass** das kosmetisch verträgliche Lösemittel aus niederen C₁-C₄-Alkoholen, Polyolen, Polyolethern, C₅-C₁₀-Alkanen, C₃₋₄-Ketonen, C₁-C₄-Alkylacetaten, Dimethoxyethan, Diethoxyethan und deren Gemischen ausgewählt ist.

18. Zusammensetzung zur kosmetischen Behandlung von Keratinmaterialien nach irgendeinem der vorhergehenden Ansprüche, **dadurch gekennzeichnet, dass** sie Zusätze umfasst, wie etwa kationische, anionische, nicht ionische oder amphotere Polymere, modifizierte oder nicht modifizierte nicht flüchtige Silikone, anionische, amphotere, zwitterionische, nicht ionische oder kationische, assoziative oder nicht assoziative natürliche oder synthetische polymere Verdickungsmittel, nicht polymere Verdickungsmittel, wie Elektrolyten, Zucker, perlglanzgebene Mittel, Trübungsmittel, Sonnenschutzfilter, Parfums, Farbstoffe, organische oder mineralische Partikel, Konservierungsmittel, pH-Stabilisatoren.

19. Verfahren zur kosmetischen Behandlung von Keratinmaterialien, **dadurch gekennzeichnet, dass** auf die Keratinmaterialien eine Zusammensetzung zur kosmetischen Behandlung nach irgendeinem der vorhergehenden Ansprüche aufgebracht wird.

20. Verwendung einer Zusammensetzung zur kosmetischen Behandlung nach irgendeinem der Ansprüche 1 bis 18 als Haarspülung.
